# EUROPEAN PATENT APPLICATION

(11) **EP 4 711 512 A2**
(43) Date of publication of application: **18.03.2026**
(21) Application number: 25226255.5
(22) Date of filing: 28.12.2021
(51) Int. Cl.: D04H 1/732

(54) **STERILIZATION WRAP AND METHODS OF FABRICATION AND USE**

(62) Divisional of application: 21847543.2
(71) Applicant: Ahlstrom Oyj, 00100 Helsinki (FI)
(72) Inventor: BARCOMB, Christopher, Windsor Locks, Connecticut, 06096 (US); BENTON, Douglas McKee, Windsor Locks, Connecticut, 06096 (US)
(74) Representative: Hoffmann Eitle

(57) **Abstract**

Disclosed herein are advantageous sterilization wraps, and related methods of fabrication and use thereof. More particularly, the present disclosure provides advantageous sterilization wraps having an outer layer comprising a thermoplastic material and an inner layer comprising a wet-laid non-woven material, with the outer layer secured and bonded to the inner layer by at least a first weld area. The present disclosure also provides for a method for fabricating a sterilization wrap including providing an outer layer comprising a thermoplastic material, and providing an inner layer comprising a wet-laid non-woven material, and securing and bonding the outer layer to the inner layer by at least a first weld area.

## Description

### FIELD OF THE DISCLOSURE

The present disclosure relates to sterilization wraps and systems/methods for fabricating and utilizing the sterilization wraps and, more particularly, to sterilization wraps having an outer layer comprising a thermoplastic material and an inner layer comprising a wet-laid non-woven material, with the outer layer secured and bonded to the inner layer by at least a first weld area.

### BACKGROUND OF THE DISCLOSURE

In general, some sterilization wraps or the like are known. For example, some sterilization wraps or the like are described and disclosed in U.S. Patent Pub. Nos. 2012/0227362 and 2018/0281344, the entire contents of each being hereby incorporated by reference in their entireties.

An interest exists for improved sterilization wraps, and related methods of fabrication and use.

These and other inefficiencies and opportunities for improvement are addressed and/or overcome by the sterilization wraps, assemblies and methods of the present disclosure.

### BRIEF SUMMARY OF THE DISCLOSURE

The present disclosure provides advantageous sterilization wraps, and systems/methods for fabricating and utilizing the sterilization wraps.

More particularly, the present disclosure provides advantageous sterilization wraps having an outer layer comprising a thermoplastic material and an inner layer comprising a wet-laid non-woven material, with the outer layer secured and bonded to the inner layer by at least a first weld area.

The above described and other features are exemplified by the following figures and detailed description.

Any combination or permutation of embodiments is envisioned. Additional advantageous features, functions and applications of the disclosed sterilization wraps, assemblies and methods of the present disclosure will be apparent from the description which follows, particularly when read in conjunction with the appended figures. All references listed in this disclosure are hereby incorporated by reference in their entireties.

### BRIEF DESCRIPTION OF THE DRAWINGS

The following figures are exemplary embodiments wherein the like elements are numbered alike.

Features and aspects of embodiments are described below with reference to the accompanying drawings, in which elements are not necessarily depicted to scale.

Exemplary embodiments of the present disclosure are further described with reference to the appended figures. It is to be noted that the various features, steps, and combinations of features/steps described below and illustrated in the figures can be arranged and organized differently to result in embodiments which are still within the scope of the present disclosure. To assist those of ordinary skill in the art in making and using the disclosed sterilization wraps, assemblies and methods, reference is made to the appended figures, wherein:
Figure 1 is a side perspective view of an exemplary sterilization wrap prior to welding, according to the present disclosure.
Figure 2 is a side perspective view of an exemplary sterilization wrap after welding, according to the present disclosure.
Figure 3 is a side cross-sectional view of the sterilization wrap of FIG. 2.
Figure 4 is a graph that demonstrates that an exemplary inner layer comprising a wet-laid non-woven material according to the present disclosure can be dried faster than a layer including spunbond-meltblown-spunbond (SMS) material.

### DETAILED DESCRIPTION OF THE DISCLOSURE

The exemplary embodiments disclosed herein are illustrative of advantageous sterilization wraps, and systems of the present disclosure and methods/techniques thereof. It should be understood, however, that the disclosed embodiments are merely exemplary of the present disclosure, which may be embodied in various forms. Therefore, details disclosed herein with reference to exemplary sterilization wraps and associated processes/techniques of assembly and use are not to be interpreted as limiting, but merely as the basis for teaching one skilled in the art how to make and use the advantageous sterilization wraps and/or alternative sterilization wraps of the present disclosure.

Disclosed herein are advantageous sterilization wraps, and related methods of fabrication and use thereof.

The present disclosure provides improved sterilization wraps, and improved systems/methods for utilizing and fabricating the sterilization wraps.

More particularly, the present disclosure provides advantageous sterilization wraps having an outer layer comprising a thermoplastic material and an inner layer comprising a wet-laid non-woven material, with the outer layer secured and bonded to the inner layer by at least a first weld area.

Conventional practice provides that some common materials used in sterilization wraps are (i) spunbond-meltblown-spunbond (SMS) material, (ii) hydrophobic wet-laid non-woven material, and (iii) crepe paper. All three technologies can have inherent advantages/disadvantages in the sterilization process. Traditional non-wovens/paper used in sterilization wrap applications can be inherently water repellent in order to meet the required barrier standards necessary for proper sterilization and maintenance of sterilization over time. The water repellent nature can be primarily achieved via the fiber type (e.g., polypropylene) or chemical treatment (e.g., fluorocarbon).

In general, sterilization wraps have two main product types, based on wrapping method - sequential wrapping and simultaneous wrapping. A large portion of the market uses simultaneous wrapping. As such, the market desires simultaneous wrap, but simultaneous wrap can generally be limited to certain conventional SMS type wraps because of ultrasonic bonding limitations of certain conventional wet-laid and crepe materials.

In general, traditional sterilization wraps are square or rectangular sheets of non-woven/paper used to wrap one or a collection of medical devices that will be sterilized following standard healthcare practices. Medical device sterilization standards typically require the use of two sheets of wrap to comply with necessary sterilization efficacy and barrier protection both at initial sterilization as well as to maintain sterilization over time.

Sterilization wraps can generally be separated into two product offerings based on the method of wrapping the medical device(s) with the required two sheets - sequential wrapping and simultaneous wrapping.

Sequential wrapping can be defined as two single sheets of non-woven/paper that should be individually wrapped around the medical device(s). The device is contained with a single sheet of wrap, and then subsequently repeated again with a second sheet. In general, all three conventional material types of sterilization wrap ((i) (SMS) material, (ii) hydrophobic wet-laid non-woven material, and (iii) crepe paper) can be used in sequential wrapping and can be combined such that the two sheets of wrap used in the sterilization system can be the same material or two different materials (e.g., wet-laid inner plus SMS outer; crepe inner plus wet-laid outer; SMS inner plus crepe outer; etc.).

Simultaneous wrapping can be defined as two sheets of non-woven that are ultrasonically bonded together along two edges for convenient wrapping of one or a collection of medical devices that will be sterilized following standard healthcare practices. The sonic bonded sheets allow the medical device(s) to be wrapped in a one-step wrapping process, versus two steps in sequential wrapping. This can reduce time and errors in the sterilization process, and can be deemed more efficient.

Due to the fact that simultaneous wrapping generally requires two wrap sheets to be sonic welded, the market predominantly utilizes SMS material since polypropylene can be highly compatible with ultrasonic bonding. Some alternative conventional materials (crepe and wet-laid) can be less compatible. Crepe paper is predominantly cellulosic and generally has very poor ultrasonic bond properties. Conventional wet-laid non-wovens sometimes used in sterilization wrap today can have modest ultrasonic bonding capability due to their synthetic fiber and binder composition.

Due to the convenience of simultaneous wrapping and efficiency generally associated to its use, sterilization wrap sold today is overwhelmingly simultaneous wrap. For example, greater than 90% of the US market is currently sold as simultaneous wrap, and generally consists primarily of SMS non-woven products.

Steam sterilization, one of the most common sterilization methods used in the healthcare industry, requires moisture to penetrate through the sterilization wrap, sterilize the medical device(s) within the wrapped container, and then exit completely out of the wrapped container. It is typically critical that all moisture exits the environment that is wrapped, therefore most sterilization standards require a minimum drying time (e.g., 20 to 30 minutes) to ensure substantially all moisture is eliminated. If the medical device is aseptically unwrapped and moisture droplets are found (called wet packs), the medical device is generally considered to be non-sterile, and the sterilization process must be repeated. The presence of wet packs is an expensive and time-consuming issue in the healthcare industry due to the requirement to re-sterilize.

In general, since the conventional layers of a wrap are typically hydrophobic/repellent, a separate absorbent trayliner layer or layers are often utilized in combination with the sterilization wrap in order to help improve the dissipation of steam moisture outside of the wrapped system. These trayliners are typically absorbent non-woven or paper placed on the bottom of a tray or directly under a tray. The trayliner attracts the steam away from the medical device and can efficiently dissipate the moisture to expedite removal from inside the wrapped container. The trayliner is often an extra step in the process, therefore the user can mistakenly forget to include it in the wrapping process, increasing likelihood of wet packs and re-sterilization, adding cost and time.

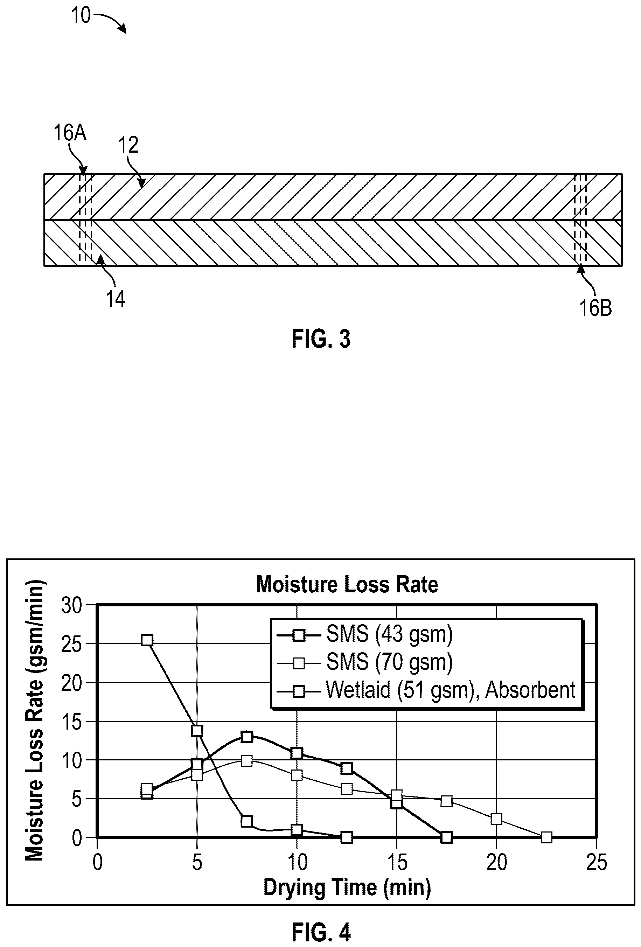

The present disclosure advantageously provides a two sheeted wrap system where an absorbent wet-laid nonwoven acts as the inner layer and an anti-static or untreated layer comprising a thermoplastic material (e.g., SMS) acts as the outer layer. In certain embodiments, the cellulosic construction of the wet-laid material provides improved and/or superior moisture absorption and wicking properties as well as improved and/or superior evaporative drying rates than hydrophilic treated polypropylene SMS material. While an absorbent treatment onto the polypropylene fibers enhances wicking, polypropylene is naturally hydrophobic and adsorptive but not absorptive and therefore its performance can have limitations. Cellulosic fibers can provide superior wicking performance and can be absorptive (water is absorbed into the cellulose structure and not only on the surface). Its capabilities and absorption capacity compared to hydrophilic treated SMS material are superior. Moisture dissipation through an exemplary wet-laid layer is better (see FIG. 4, and further discussed below). The absorptive and adsorptive properties of the exemplary absorbent wet-laid layer make it superior to absorbent treated SMS, and its water absorbent capacity can allow for the elimination of the trayliner in the sterilization process.

In FIG. 4, this graph demonstrates that an exemplary wet-laid layer according to the present disclosure can be dried faster than a SMS layer. The three samples used are: (1) a 43 gsm polypropylene based SMS (commercial grade with antistatic treatment and inherently hydrophobic); (2) a 70 gsm polypropylene based SMS (commercial grade with antistatic treatment and inherently hydrophobic); and (3) a 51 gsm exemplary wet-laid without water repellency treatment (no wax and fluorocarbon treatment) with additional Aerosol OT (A-OT) for water absorbency.

0.5 g of water was applied to 7.1 in² sample of each materials. The samples were then dried at 100°C. The rate of water loss was measured at different time intervals. The data obtained shows that the exemplary wet-laid has a faster rate of evaporation and therefore dries at a faster rate compared to the SMS samples. Without wishing to be bound by theory, it is noted that the exemplary wet-laid dried faster because the addition of the surfactant aids wicking of moisture (capillary action), and also because the capacity for water removal is much higher with cellulose because cellulose allows absorption (absorbed into the fiber). SMS with its polypropylene fibers only provides surface wetting adsorption.

Some current conventional sterile wrap materials have some level of water repellency for good barrier performance and sterility. But this water repellency can lower the efficiency of moisture dissipation from a wrapped device (during/after sterilization), which can lead to wet packs. Previously, water absorbent trayliners can be added as an additional (separate) layer or layers, with the wraps by customer during end-use; but this can create inefficiency and/or risks for error. Alternatively, a typically-repellent SMS layer could be treated to become more absorbent, but SMS generally cannot replicate the absorbent properties of a cellulosic nonwoven (due to the inherent hydrophobic character of polypropylene fibers).

The present disclosure advantageously provides a sterilization wrap where the inner layer provides a moisture absorbent character in addition to sterile barrier properties. In certain embodiments, the sterilization wraps of the present disclosure use a wet-laid material as the inner layer of the wrap, to provide the wrap with the necessary absorbency.

As noted above, simultaneous-wrap systems are the preferred type of sterilization wraps by customers. It is noted that a product for simultaneous-wrap can require sonic bonding between the inner/outer layers (e.g., along two edges of the sheets). In general, sonic bonding is not typically a problem between the typical SMS/SMS configurations, but adequate sonic bonding is typically not very feasible for combining conventional SMS/wet-laid sheets. The present disclosure advantageously provides a wet-laid layer having enhanced ultrasonic bond capability, in order to more readily provide for a sonic-bonded two-sheet thermoplastic material (e.g., SMS)/wet-laid sterilization wrap for use in simultaneous wrapping (e.g., to use a wet-laid inner layer having certain synthetic binder and fiber content, as discussed further below).

This advantageous hybrid two sheet wrapping system of an absorbent wet-laid non-woven material plus a thermoplastic material (SMS) layer can advantageously accomplish at least the following: (1) improved commercial robustness and viability for a sonic-bond simultaneous wrap system that contains wet-laid as at least one layer; (2) allows the user to eliminate a trayliner in the sterilization process, increasing convenience and efficiency of the overall sterilization system; (3) faster drying times in steam sterilization cycles, reducing sterilization process costs and turnaround times - also reduced wet-pack generation and re-sterilization needs; (4) maintains the necessary strength and barrier property requirements.

Referring now to the drawings, like parts are marked throughout the specification and drawings with the same reference numerals, respectively. Drawing figures are not necessarily to scale and in certain views, parts may have been exaggerated for purposes of clarity.

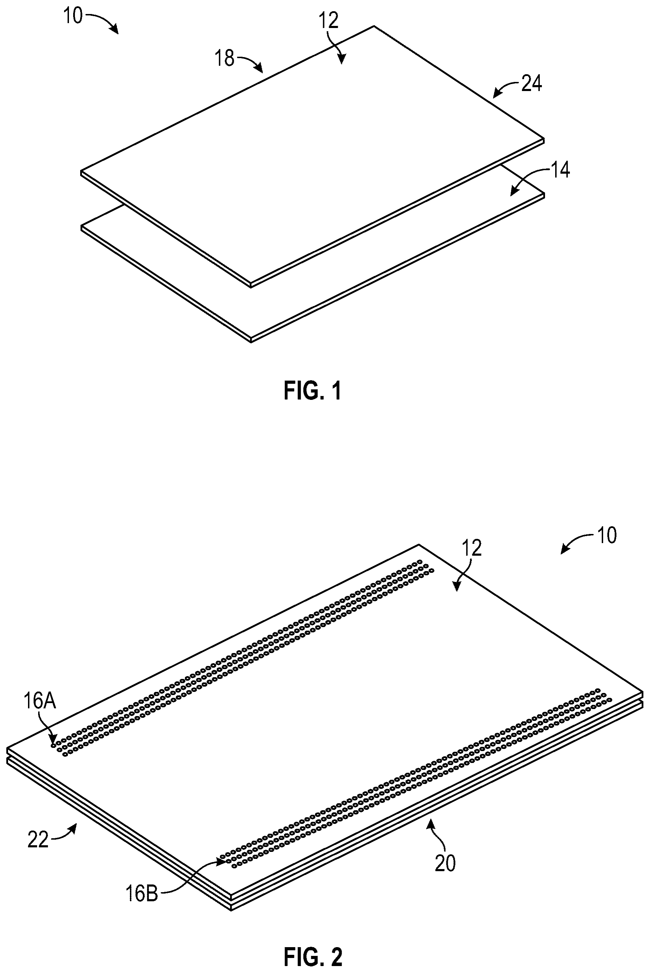

As shown in FIG. 1, there is illustrated a side perspective view of an exemplary sterilization wrap 10 prior to welding. In general and as discussed further below, exemplary sterilization wrap 10 includes an outer layer 12 comprising a thermoplastic material, and an inner layer 14 comprising a wet-laid non-woven material, with the outer layer 12 secured and bonded to the inner layer 14 by at least a first weld area 16A (FIGS. 2 and 3).

FIG. 2 depicts a side perspective view of an exemplary sterilization wrap 10 after welding. FIG. 3 is a side cross-sectional side view of an exemplary sterilization wrap 10 after welding.

The present disclosure further provides for a method for fabricating a sterilization wrap 10 including providing an outer layer 12 comprising a thermoplastic material, and providing an inner layer 14 comprising a wet-laid non-woven material, and securing and bonding the outer layer 12 to the inner layer 14 by at least a first weld area 16A.

In exemplary embodiments, the outer layer 12 is secured and bonded to the inner layer 14 by at least a first weld area 16A and by at least a second weld area 16B. In general, weld areas 16A, 16B provide a weld or bond area 16A, 16B through the outer and inner layers 12, 14.

In some embodiments, the weld areas 16A, 16B are ultrasonic weld areas (e.g., an ultrasonic weld or bond area 16A, 16B through the outer and inner layers 12, 14), although the present disclosure is not limited thereto. It is noted that other welding techniques and methods can be utilized to secure and bond the outer layer 12 to the inner layer 14.

Each weld area 16A, 16B can include at least one continuous and/or non-continuous welding line and/or welding spot. In some embodiments, each weld area 16A, 16B includes a plurality of continuous and/or non-continuous welding lines and/or welding spots.

It is noted that outer layer 12 can be secured and bonded to the inner layer 14 by at least one weld area 16A (and/or 16B) utilizing a variety of welding lines and/or welding spots, along a variety of locations and/or positions of outer layer 12 and inner layer 14. In general, it is noted that the outer layer 12 and the inner layer 14 are each free of an added adhesive material after being secured and bonded to one another. Stated another way, the outer layer 12 and inner layer 14 can be assembled (secured and bonded to one another) without the use of an additional adhesive material (but with the binder present in the inner layer 14). For example, layers 12, 14 can be assembled/secured together (with the binder present in the inner layer 14) without the use of an additional adhesive material, such as, for example, without non-binder/dispersion types of materials that can take the form of glues, tapes, webs, sprays, foams that utilize hot melt adhesives, pressure sensitive adhesives, curable adhesives (e.g., polyurethane and others), and also without thermoplastic adhesives (e.g., ethyl vinyl acetates, ethylene-vinyl alcohol copolymers, and ionomers), or without other conventional industrial adhesive chemistries.

For example, the outer layer 12 can be secured and bonded to the inner layer 14 by at least the first weld area 16A along a first edge 18 of the outer and inner layers 12, 14.

As shown in FIG. 2, the outer layer 12 can be secured and bonded to the inner layer 14 by at least a second weld area 16B along a second edge 20 of the outer and inner layers 12, 14.

It is noted that weld areas 16A and/or 16B (or additional weld areas 16) can be positioned along edges 22 and/or 24 of the outer and inner layers 12, 14. Again, it is noted that outer layer 12 can be secured and bonded to the inner layer 14 by at least one weld area 16A (and/or 16B) utilizing a variety of welding lines and/or welding spots, along a variety of locations and/or positions of outer layer 12 and inner layer 14.

In exemplary embodiments, the outer layer 12 is directly secured and directly bonded to the inner layer 12 by at least the first weld area 16A. In some embodiments, the outer layer 12 is secured and bonded only and solely to the inner layer 14 by at least the first weld area 16A.

In some embodiments, the outer layer 12 defines a thickness of thermoplastic material, and the inner layer 14 defines a thickness of wet-laid non-woven material; and the outer layer 12 is not secured or bonded to another layer or thickness of material after the outer layer 12 is secured and bonded to the inner layer 14 by at least the first weld area 16A.

In certain embodiments, the outer layer 12 secured and bonded to the inner layer 14 by at least the first weld area 16A has a bond strength greater than or equal to 80 cN/15 mm, preferably greater than or equal to 100 cN/15 mm, according to the standard method ASTM F88.

In exemplary embodiments, the outer layer 12 comprises a non-woven melt-blown material, preferably wherein the outer layer 12 comprises melt-blown synthetic fibers, more preferably wherein the outer layer 12 comprises spunbond-meltblown-spunbond (SMS) synthetic fibers, even more preferably wherein the outer layer 12 comprises SMS polyolefin fibers, such as SMS polypropylene fibers.

In general, the basis weight of exemplary outer layer 12 is from about 35 grams per square meter (gsm) to about 85 gsm, and optionally is treated with an anti-static composition. The outer layer 14 can contribute to bacterial barrier properties of wrap 10.

In exemplary embodiments, the basis weight of the inner layer 14 is from about 35 grams per square meter (gsm) to about 85 gsm.

Exemplary inner layer 14 is absorbent and moisture wicking. In certain embodiments, the wet-laid non-woven material of the inner layer 14 comprises cellulose fibers, synthetic fibers, binder, and optionally a surfactant.

In general, the inner layer 14 comprises cellulose fibers in a quantity between 40% to 100% by weight based on the dry weight of the fibers in the inner layer 14, preferably between 60% to 85% by weight based on the dry weight of the fibers in the inner layer 14.

The cellulose fibers of the inner layer can comprise at least one of hardwood pulp fibers, softwood pulp fibers, annual plant fibers or cotton fibers. The inner layer 14 can comprise at least one of mercerized or un-mercerized cellulosic fibers, or a combination thereof. The fibers can be mercerized and/or un-mercerized cellulosic fibers. Mercerization adds extra bulk to the fibers for increased absorption.

In some embodiments, the wet-laid non-woven material of the inner layer 14 is micrexed or micro-creped (e.g., micrexing or micro-creping of the wet-laid non-woven material of the inner layer 14). The wet-laid is optionally and preferably micrexed. This aids drapability, softness, barrier properties and absorption capacity.

In some embodiments, the inner layer 14 comprises synthetic fibers, preferably polyester staple fibers, more preferably polyethylene terephthalate (PET) fibers. In certain embodiments, the inner layer 14 comprises man-made cellulosic fibers (e.g., Lyocell or Rayon).

For example, the inner layer 14 can comprise synthetic fiber in a quantity between 5% to 60% by weight synthetic fibers, preferably 15% to 30%, the percentage based on the dry weight of the fibers of the inner layer 14, and optional synthetic binder fibers are included in this percentage, if present.

In exemplary embodiments, the inner layer 14 comprises a binder, the binder comprising acrylic dispersions having acrylonitrile, preferably comprising at least one of anionic acrylate-styrene and/or anionic acrylate-styrene-acrylonitrile.

For example, the inner layer 14 can comprise binder in a quantity between 15 wt% to 40 wt%, preferably 15 to 30 wt%, more preferably 18 to 28 wt%, even more preferably 20 to 25 wt%, the weight percentage based on the total dry weight of the inner layer 14.

In some embodiments, the inner layer 14 comprises a surfactant, the surfactant preferably an anionic surfactant, preferably an anionic surfactant such as ammonium lauryl sulfate, sodium laureth sulfate, sodium lauryl sarcosinate, sodium myreth sulfate, sodium pareth sulfate, sodium stearte, sodium lauryl sulfate, α olefin sulfonate, and ammonium laureth sulfate, more preferably sodium bis(2-ethyl-1-hexyl) sulfosuccinate.

For example, the inner layer 14 can comprise a surfactant in a quantity between 0.5 to 5%, preferably between 1 to 3%, more preferably between 1 to 2%, of a binder mixture.

Table 1 below provides a summary of exemplary components/materials and exemplary amounts of components/materials for the wet-laid non-woven material of the inner layer 14.

In general, the total basis weight of the sterilization wrap 10 can be from about 70 grams per square meter (gsm) to about 170 gsm.

The exemplary sterilization wrap 10 is suitable for use to sterilize a medical device utilizing the simultaneous wrap technique during a sterilization process. The exemplary sterilization wrap 10 is suitable for use to sterilize a medical device without utilizing an additional trayliner during a sterilization process.

In general, the sterilization wrap 10 is permeable to a sterilizing agent, such as steam. The exemplary sterilization wrap 10 exhibits microbial barrier properties and/or is impermeable to bacteria. In certain embodiments, the sterilization wrap 10 meets the requirements of European standard EN 868 Part 2 as a sterile barrier system. In some embodiments, the sterilization wrap 10 meets the requirements of international standard ISO 11607 Part 1 as a sterile barrier system.

As such and as discussed above, the present disclosure advantageously provides a two sheeted wrap 10 or wrap system 10 where an absorbent wet-laid nonwoven layer 14 acts as the inner layer 14 and an anti-static or untreated layer 12 comprising a thermoplastic material (e.g., SMS) acts as the outer layer 12. The cellulosic construction of the wet-laid material 14 provides improved and/or superior moisture absorption and wicking properties as well as improved and/or superior evaporative drying rates than hydrophilic treated polypropylene SMS material. Cellulosic fibers can provide superior wicking performance and can be absorptive. The absorptive and adsorptive properties of the exemplary absorbent wet-laid layer 14 make it superior to absorbent treated SMS, and its water absorbent capacity can allow for the elimination of the trayliner in the sterilization process.

The present disclosure also advantageously provides a sterilization wrap 10 where the inner layer 14 provides a moisture absorbent character in addition to sterile barrier properties. The sterilization wraps 10 of the present disclosure use a wet-laid material 14 as the inner layer 14 of the wrap 10, to provide the wrap 10 with the necessary absorbency.

The present disclosure also advantageously provides a wet-laid layer 14 having enhanced ultrasonic bond capability, in order to more readily provide for a sonic-bonded two-sheet thermoplastic material 12 (e.g., SMS)/wet-laid sterilization wrap 10 for use in simultaneous wrapping (e.g., to use a wet-laid inner layer 14 having certain synthetic binder and fiber content, as discussed above.

This advantageous hybrid two sheet wrap 10 and wrapping system of an absorbent wet-laid non-woven material 14 plus a thermoplastic material (e.g., SMS) layer 12 can advantageously accomplish at least the following: (1) improved commercial robustness and viability for a sonic-bond simultaneous wrap 10 and wrap system that contains wet-laid as at least one layer 14; (2) allows the user to eliminate a trayliner in the sterilization process, increasing convenience and efficiency of the overall sterilization system; (3) faster drying times in steam sterilization cycles, reducing sterilization process costs and turnaround times - also reduced wet-pack generation and re-sterilization needs; (4) maintains the necessary strength and barrier property requirements of the wrap 10.

The present disclosure advantageously provides for a sterilization wrap 10 comprising two layers 12, 14, which can be joined along two edges 18, 20 to provide a wrap 10 or wrap system 10 suitable for use in the simultaneous wrap technique. An exemplary inner layer 14 of the wrap/system 10 exhibits moisture absorbent characteristics, improving the moisture loss rate (drying rate) during steam sterilization cycles, and thereby reducing the prevalence of wet-pack formation. The inner layer 14, which provides this absorbency, can comprise a wet-laid non-woven material. The outer layer 12 of the wrap/system 10 provides the wrap 10 with the necessary strength characteristics, and can comprise a polyolefin SMS non-woven or the like. The layers 12, 14 can be joined together along one or more edges 18, 20 of the wrap/system 10 by means of sonic bonding or the like, which is enabled through use of the improved wet-laid non-woven material of the inner layer 14.

This disclosure is further illustrated by the following examples, which are non-limiting.

### EXAMPLES

Different binder polymers were tested.

In order to improve the moisture absorbency/moisture wicking, the samples had no water repellency treatment such as wax and fluorocarbon treatment. A cellulosic wet-laid containing only Hycar 26430x6 binder, Sample 4, serves as a reference.

The samples according to the present disclosure were optimized for a combination of properties: strength, softness/flexibility, and absorbency.

Desirably, the cellulosic wet-laid would have suitable mechanical properties, would be as soft as possible, and would also be highly absorbent. However, these properties in many ways can work against each other.

The samples had the following base web formulation: 75 wt% Northern Bleached Softwood Kraft (NBSK) pulp of total fibers; 25 wt% of Teijin PET stable fibers (1.7 dtex and 15 mm) of total fibers.

Samples 1, 1.1, 1.2, 1.3 and 1.4 contained the above base web formulation and contained varying amounts of binder Acronal S728 commercialized by BASF and of surfactant Aerosol OT-75 commercialized by Solvay.

Samples 2, 2.1, 2.2, 2.3 and 2.4 contained the above web formulation and contained varying amounts of binder Acronal S504 commercialized by BASF and of surfactant Aerosol OT-75.

Sample 3 contained the above web formulation and contained a mixture with equal amounts of Acronal S728 and S504 and of Aerosol OT-75.

Sample 4 contained the above base web formulation and contained the Hycar 26430x6 binder, and Sample 4.1 contained the above base web formulation and the surfactant Aerosol OT-75.

Comparative 2.1 and 2.2 were polypropylene based SMS according to the conventional art of 70 and 43 g/m² respectively.

**Table 1: Type of binder and amount thereof:**

| | **Binder type** | **Binder quantity of the wetlaid** | **Aerosol OT surfactant quantity in binder mixture** |
|---|---|---|---|
| **Sample 1** | Acronal S 504 | 24% | - |
| **Sample 1.1** | Acronal S 504 | 24% | 1% |
| **Sample 1.2** | Acronal S 504 | 24% | 2% |
| **Sample 1.3** | Acronal S 504 | 20% | 2% |
| **Sample 1.4** | Acronal S 504 | 30% | 2% |
| **Sample 2** | Acronal S 728 | 24% | - |
| **Sample 2.1** | Acronal S 728 | 24% | 1% |
| **Sample 2.2** | Acronal S 728 | 24% | 2% |
| **Sample 2.3** | Acronal S 728 | 20% | 2% |
| **Sample 2.4** | Acronal S 728 | 30% | 2% |
| **Sample 3** | Acronal S 728 + Acronal S 504 | 24% | - |
| **Sample 4** | Hycar 26430x6 | 24% | - |
| **Sample 4.1** | Hycar 26430x6 | 24% | 2% |

The addition of A-OT helps a lot in one way, but can hurts in others. On the positive, it significantly improves absorbency properties. On the negative, the aggressive nature of anionic surfactant tends to penetrate deeply into fibers (particularly cellulosic) impacting fiber integrity. It can reduce wet tensile strength/durability of nonwovens. As shown below on Table 2 when A-OT is added, strength declined.

Also, glass transition temperature (Tg) of the binder can be important for imparting flexibility making for a more efficient/productive wrapping process of instrument trays. It can also impact ultrasonic bonding performance. Acronal S 504 has a Tg of 4°C and Acronal S 728 has a Tg of 23°C.

A lower Tg for the binder tends to make the nonwoven softer.

Also, a lower Tg makes the binder more heat processable, which can improve welding performance, notably ultrasonic bonding properties.

However, lower Tg polymers often have lower strength properties - particularly dry strength.

It is noted that acrylonitrile is often added to a polymer backbone to impart toughness. This is important when considering that these binder systems are not self-crosslinking, so wet/chemical resistance should come from the polymer backbone.

Acrylonitrile based copolymers are known to be more robust - particularly in terms of chemical resistance. They can be useful for applications where solvent resistance is needed.

The inventors noticed that the use of a binder comprising acrylonitrile could counter-balance the aggressive nature of the A-OT in the formulation - allowing the non-woven to maintain adequate strength properties, both in terms of dry and wet strength.

Also, the acrylonitrile is not a large enough component of the polymer backbone to negatively impact sonic bond. One can still get the benefit of the very soft, thermoplastic, butyl acrylate backbone.

**Table 2: Mechanical properties:**

| | **Basis weight (g/m²)** | **TA2 Thickness (µm)** | **X grab Tensile (grams)** | **X wet Tensile (grams)** | **X Trap Tear (grams)** | **Dry Mullen (g/cm²)** |
|---|---|---|---|---|---|---|
| **Sample 1** | 49 | 142 | 11083 | 2481 | 77 | 1900 |
| **Sample 1.1** | 56 | 178 | 10272 | 2212 | 111 | 2133 |
| **Sample 1.2** | 56 | 184 | 10733 | 1998 | 101 | 2267 |
| **Sample 1.3** | 55 | 181 | 10433 | 1601 | 117 | 2117 |
| **Sample 1.4** | 62 | 185 | 12100 | 2073 | 92 | 2700 |
| **Sample 2** | 51 | 157 | 12733 | 2755 | 78 | 2083 |
| **Sample 2.1** | 55 | 188 | 11332 | 2061 | 92 | 2083 |
| **Sample 2.2** | 49 | 172 | 10113 | 1660 | 108 | 1900 |
| **Sample 2.3** | 52 | 174 | 11781 | 2155 | 97 | 2467 |
| **Sample 2.4** | 57 | 189 | 11616 | 1866 | 125 | 2067 |
| **Sample 3** | 58 | 183 | 12214 | 1644 | 101 | 2467 |
| **Sample 4** | 52 | 169 | 10713 | 2534 | 89 | 2100 |
| **Sample 4.1** | 52 | 180 | 10300 | 2038 | 87 | 2133 |

**Table 3: Absorbency properties:**

| | **Absorption Capacity (%)** | **Water Drop (s)** | **Improvement to absorption rate (%)** | **MVTR (g/m²/day)** |
|---|---|---|---|---|
| **Sample 1** | 81.5 | 300.0 | 0% | - |
| **Sample 1.1** | 243.5 | 17.7 | 94% | 1703.0 |
| **Sample 1.2** | 232.6 | 12.3 | 96% | 1690.0 |
| **Sample 1.3** | 253.3 | 11.7 | 96% | 1889.5 |
| **Sample 1.4** | 217.2 | 11.5 | 96% | 1889.5 |
| **Sample 2** | 222.7 | 133.7 | 55% | - |
| **Sample 2.1** | 255.8 | 4.0 | 99% | 1892.7 |
| **Sample 2.2** | 270.1 | 2.2 | 99% | 1758.0 |
| **Sample 2.3** | 264.7 | 1.4 | 100% | 1932.8 |
| **Sample 2.4** | 232.3 | 2.0 | 99% | 1926.4 |
| **Sample 3** | 245.1 | 6.3 | 98% | 1990.5 |
| **Sample 4** | 110.3 | 300.0 | 0% | 1966.0 |
| **Sample 4.1** | 180.8 | 300.0 | 0% | - |
| **Comparative 2.1** | 60.2 | 300.0 | N/A | 1861.0 |
| **Comparative 2.2** | 93.5 | 300.0 | N/A | 1854.0 |

**Table 4: Bond strength properties:**

| | **LW Bond Strength (g)** | **HW Bond Strength (g)** | **LW x HW bond performance** |
|---|---|---|---|
| **Sample 1** | 613 | 527 | 61% |
| **Sample 1.1** | 666 | 371 | 50% |
| **Sample 1.2** | 383 | 90 | 28% |
| **Sample 1.3** | 592 | 377 | 44% |
| **Sample 1.4** | 335 | 351 | 33% |
| **Sample 2** | 776 | 546 | 89% |
| **Sample 2.1** | 758 | 540 | 83% |
| **Sample 2.2** | 675 | 601 | 78% |
| **Sample 2.3** | 696 | 568 | 72% |
| **Sample 2.4** | 703 | 437 | 56% |
| **Sample 3** | 648 | 545 | 67% |
| **Sample 4** | 752 | 783 | 94% |
| **Sample 4.1** | 432 | 484 | 39% |

### Comments on Tables 2 and 3:

In order to minimize wet pack formation and improve the drying time of sterilization wraps, the wax and fluorocarbon treatment were removed, so the water absorbency could improve.

The water absorbency reached with the Hycar 26430x6 was not entirely satisfactory, so different binder compositions were tested to further improve water absorbency.

Properties of the wet-laid used to evaluate the performance of a binder within the cellulosic wet-laid were: strength/durability; softness/flexibility; and absorbency/moisture wicking.

To further improve the water absorbency of the wet-laid media, additional surfactant was added. However, adding a surfactant had a detrimental effect on certain mechanical properties of the wet-laid media, notably on the wet tensile strength. The different mechanical properties presented on Table 2 were measured following test methods described in the Test Methods below. The addition of a surfactant reduced the wet tensile strength of all samples. However, the impact was somewhat moderate on samples containing the Acronal S 504 binder.

It is believed that this binder is less affected because Acronal S 504 has acrylonitrile added to the polymer backbone and this confers a certain chemical resistance. This chemical resistance counter-balances the aggressive nature of the surfactant added to the binder.

For a sterilization wrap, among the different mechanical properties measured, wet tensile strength can be of importance because a preferred sterilization method is steam sterilization. The other mechanical properties were less affected by the addition of a surfactant and show some variation but remain at an acceptable level.

### Comments on Table 3:

As indicated above, one of the key properties for selecting the binder was the water absorbency and moisture wicking. In order to evaluate these properties, water absorption capacity and water drop value were measured (test methods are described below). The improvement of absorption rate was derived from the water drop measurement using Sample 4 as the reference.

This data shows that Acronal S 504 does not really improve the absorbency properties when used alone. However, Acronal S 728 seems to have inherent water absorbency properties. When a surfactant was introduced in the binder in samples comprising Acronal S 504 and Acronal S 728 the absorbency properties improved substantially. The surfactant had very limited impact on Sample 4.1 comprising Hycar 26430x6.

Even though Acronal S 728 seems to have better water absorbency, as discussed above the surfactant seems to affect more the wet strength properties of wet-laid medias comprising the Acronal S 728 binder relative to wet-laid medias comprising the Acronal S 504 binder.

### Comments on Table 4:

In order to evaluate the bonding performance of the samples, each sample was heat-sealed on its own and a bond strength test was performed according to the test protocol described in ASTM F88.

All samples tested showed a relatively good bond strength performance. This data shows for wet-laid medias, optimal binder content can be between 15 and 30 wt%, and preferably between 18 and 28 wt%. If the amount of binder is very low there will not be sufficient material to achieve acceptable level of bonding. If the amount of the binder is very high, then it tends to reduce the contribution of the fiber to the bonding thus reducing the overall bond strength of the wet-laid.

### Ultrasonic Bonding With SMS

Based on these initial findings, an exemplary recipe was further tested for ultrasonic bonding performance and had the following recipe:

Base fiber mix: 75 wt% of NBSK pulp and 25 wt% of Teijin PET stable fibers (1.7 detx and 15 mm) (wt% per total fibers).

Binder mix: 97% of Acronal S 504, 2 wt% of Aerosol OT, 1 wt% green pigments (optional for coloring) and ammonia was used to adjust the pH at 8. The binder mix represented 22 wt% per total dry weight of the wet-laid.

Different samples with different basis weight were prepared and assembled to a polypropylene based SMS layers with ultrasonic bonding. The ultrasonic bonding parameters on an Aurizon instrument were: 4.5 mm wide 0.015" x 0.015" deep bond and knurled pattern anvil using settings of 55 psi for double pattern anvil and 35 psi single anvil, at 100% amplitude, and an operation speed of 100-350 fpm.

The samples were prepared as follows: (1) Sample X1.1 => Outer 43 gsm SMS blue/Inner 37 gsm wetlaid green; (2) Sample X1.2 => Outer 43 gsm SMS blue/ Inner 46 gsm wetlaid green; and (3) Sample X1.3 => Outer 70 gsm SMS blue/ Inner 63 gsm wetlaid green.

The bond strength between the wet-laid layer and the SMS layer for sample X1.1 and sample X1.3 was measured according to the standard method ASTM F88. The bond strengths for sample X1.1 and sample X1.3 were respectively 232 and 237 cN/15mm.

Advantageously and surprisingly, it was found that it is possible to bond a wet-laid layer to an SMS layer at a bond strength greater than or equal to 100 CN/15 mm.

**Table 5: - Test Methods: The different properties in Tables 2-4 were measured following the Test methods listed below:**

| Property, unit | Test method |
|---|---|
| Basis weight, gsm | ASTM D3776 |
| TA2 Thickness, microns | Tappi T411 |
| MD Grab Tensile, grams | ASTM D5034 |
| CD Grab Tensile, grams | ASTM D5034 |
| MD Trap Tear, grams | ASTM D4533 |
| CD Trap Tear, grams | ASTM D4533 |
| MD Dry Tensile Strength, grams | Tappi T494 |
| CD Dry Tensile Strength, grams | Tappi T494 |
| Dry Mullen, g/cm2 | Tappi T403 |
| Absorption Capacity (%) | EDANA NWSP 10.1 |
| Water drop (sec) | TAPPI T-432 OM-94 |
| Bond Strength (g) | ASTM F88 |
| MVTR | ASTM E96 |

In Table 2, the X values are derived from MD and CD measurements as follows: (1) X Grab tensile = Square root ( MD Grab tensile * CD Grab tensile); (2) X Wet tensile = Square root ( MD Wet tensile * CD Wet tensile); and (3) X Trap tear = Square root (MD Trap tear * CD Trap tear).

In Table 3, improvement to absorption rate was calculated using Sample 4 as reference and using water drop measurements.

### Dry Time Validation Study: Pre-Vacuum Steam:

Dry Time Testing was completed on the following sterilization wrap design conditions: (1) Sample X1.4 => Outer 43 gsm SMS blue/ Inner 40 gsm wetlaid green; and (2) Sample X1.5 => Outer 70 gsm SMS blue/ Inner 62 gsm wetlaid green.

In pre-vacuum steam sterilization, the medical instrument tray and all accessories must be completely free of moisture post sterilization. Moisture could provide a vector for micro-organisms to enter a closed containment system and likely contaminate the contents within that enclosed system. There are many variables that can cause a sterilization containment system to retain moisture and the establishment of time required to completely dry the wrapped medical instrument tray is important to minimum the risk of developing "wet packs" following a sterilization process. Wet packs are an industry understood term for the presence of moisture in the wrapped instrument tray, invalidating the sterility of the items found within the tray.

Sample X1.4 and Sample X1.5 were tested via a standard dry time study that is commonly available at commercial sterilization validation laboratories such as Highpower Laboratories or Nelson Laboratories. The study determines the proper drying time of the sterilization wrap when processed in a steam pre-vacuum sterilization cycle. An instrument tray is loaded with simulated medical instruments to a pre-determined weight and processed in a steam pre-vacuum cycle of 132 °C for four (4) minutes of exposure and dried for a pre-determined period of time. Following the cycle completion, the instrument tray is re-weighed and pre- and post- sterilization weights are compared and evaluated. Additionally, the instrument tray, contents and sterilization wrap are assessed for visual moisture retention. A common component found inside the wrapped system is the inclusion of a trayliner, an absorbent sheet that is placed on the bottom of the instrument tray or directly under the instrument tray. The trayliner acts as an absorbent extension of the overall system to enhance the moisture wicking properties of the system and improve drying performance (and reduce instances of wet-packs). For the samples X1.4 and Samples X1.5, trayliners were not included in the wrapped system.

The results of the drying time study were the following:

Sample X1.4 was validated for 12 minutes drying time with a 9 lb tray of instruments and no trayliner. For comparison a commercial simultaneous wrap (two sheets of polypropylene based SMS, 50 gsm each) had a validated drying time of 20 minutes for a similar 9 lb tray of instruments with a trayliner. A 40% reduction in drying time at the same instrument weight and no trayliner.

Sample X1.5 was validated for 20 minutes drying time with a 25 lb tray of instruments and no trayliner. For comparison a commercial simultaneous wrap (two sheets of polypropylene based SMS, 85 gsm each) had a validated drying time of 30 minutes for a similar 24 lb tray of instruments with a trayliner. A 33% reduction in drying time at the same instrument weight and no trayliner.

### Barrier Property Testing:

### Barrier Test #1: Pre-Vacuum Steam Sterilization Validation via Half-Cycle Study:

Sample X1.4 and Sample X1.5 were tested for barrier property performance via half-cycle sterilization validation testing utilized the newly validated drying times referenced above. Half-cycle sterilization validation is a commonly available study at commercial sterilization validation laboratories such as Highpower Laboratories or Nelson Laboratories. The intent of the study was to determine the sterilization efficacy of a sterilization wrap loaded with simulated medical instruments that was inoculated with a minimum quantity of biological spores in difficult to sterilize locations of the medical instruments and processed in a pre-vacuum sterilization cycle for half the intended exposure time. The test assumes a worst-case sterilization environment which provides high probability of barrier performance if the sterilization wrap is found to eradicate the biological sports under the test conditions. For this study, the temperature remained the same (132 °C) but the exposure time was reduced from 4 minutes to 2 minutes.

### The results of the half-cycle study was the following:

Sample X1.4
Pre-Vac Steam Condition (132°C, 2 minutes exposure time) 50% exposure time reduction
Drying Time (12 minutes)
Instrument Tray Weight: 9 lbs
Test Result: All biological indicator test samples were negative for growth, all inoculated biological spores were killed
Sample X1.5
Pre-Vac Steam Condition (132°C, 2 minutes exposure time) 50% exposure time reduction
Drying Time (20 minutes)
Instrument Tray Weight: 25 lbs
Test Result: All biological indicator test samples were negative for growth, all inoculated biological spores were killed

The study demonstrated that both the high and low basis weight versions of the exemplary wrap had effective sterilization efficacy with reduced drying times.

### Barrier Test #2: Microbial Aerosol Challenge Testing:

Sample X1.4 and Sample X1.5 were also tested for additional barrier property performance via a microbial aerosol challenge test. Microbial aerosol challenge testing is an industry recognized test at commercial sterilization validation laboratories such as Highpower Laboratories or Nelson Laboratories. The intent of the test is to determine the sterilization wraps ability to maintain sterile packaging integrity following an aerosol challenge of biological spores. A wrapped medical instrument tray was prepared with Samples X1.4 and X1.5 at desired instrument tray weights for each. The wrapped trays were then processed via a standard steam pre-vacuum cycle of 132°C with a four (4) minute exposure time and drying times specific to the sample. After completion of the pre-vac sterilization process, the wrapped instrument tray system was showered with an aerosol concentration of biological spores, challenging the permeability of the wrap to microorganisms. After 30 mins of aerosol challenge, the package was aseptically opened and the contents tested for biological presence.

### The results of the half-cycle study was the following:

Sample X1.4
Pre-Vac Steam Condition (132°C, 4 minutes exposure time)
Drying Time (12 minutes)
Instrument Tray Weight: 9 lbs
Test Result: Passed
Sample X1.5
Pre-Vac Steam Condition (132°C, 2 minutes exposure time) 50% exposure time reduction Drying Time (20 minutes)
Instrument Tray Weight: 25 lbs
Test Result: Passed

**Table 6:**

| | Comparative example (two 50 gsm PP SMS layers) | Hybrid Wrap Sample #1.4 (Low BW version (Compare to two 50 gsm PP SMS layers) | Comparative example (two 85 gsm PP SMS layers) | Hybrid Wrap Sample #1.5 (High BW version (compare to two 85 gsm PP SMS layers) |
|---|---|---|---|---|
| Basis Weight, gsm (2 sheet system) | 100 | 83 | 170 | 132 |
| Pre-Vac Steam Dry Time Testing | 20 minutes | 12 minutes | 30 minutes | 20 minutes |
| Instrument Tray Load Weights (Used for all testing on this table) | 9 pounds | 9 pounds | 25 pounds | 25 pounds |
| Half-Cycle Sterilization Validation Testing (Pre-Vac Steam, 132°C, 2 minutes exposure) - Utilizing above validated dry times and instrument load weights for each grade | Pass | Pass | Pass | Pass |
| Microbial Aerosol Challenge Testing (Pre-Vac Steam, 132°C, 4 minutes exposure) - Utilizing above validated dry times for each grade | Pass | Pass | Pass | Pass |

This disclosure further encompasses the following aspects.

Aspect 1. A sterilization wrap comprising an outer layer comprising a thermoplastic material; and an inner layer comprising a wet-laid non-woven material; wherein the outer layer is secured and bonded to the inner layer by at least a first weld area.

Aspect 2. The sterilization wrap of Aspect 1, wherein the outer layer is directly secured and directly bonded to the inner layer by at least the first weld area.

Aspect 3. The sterilization wrap of any one or more of the preceding aspects, wherein the outer layer defines a thickness of thermoplastic material, and the inner layer defines a thickness of wet-laid non-woven material; and wherein the outer layer is not secured or bonded to another layer or thickness of material after the outer layer is secured and bonded to the inner layer by at least the first weld area.

Aspect 4. The sterilization wrap of any one or more of the preceding aspects, wherein the outer layer is secured and bonded to the inner layer by at least the first weld area along a first edge of the outer and inner layers.

Aspect 5. The sterilization wrap of Aspect 4, wherein the outer layer is secured and bonded to the inner layer by at least a second weld area along a second edge of the outer and inner layers.

Aspect 6. The sterilization wrap of any one or more of the preceding aspects, wherein the outer layer and the inner layer are each free of an added adhesive material.

Aspect 7. The sterilization wrap of any one or more of the preceding aspects, wherein the outer layer comprises a non-woven melt-blown material, preferably wherein the outer layer comprises melt-blown synthetic fibers, more preferably wherein the outer layer comprises spunbond-meltblown-spunbond (SMS) synthetic fibers, even more preferably wherein the outer layer comprises SMS polyolefin fibers, such as SMS polypropylene fibers.

Aspect 8. The sterilization wrap of any one or more of the preceding aspects, wherein at least one of the first weld area or the second weld area is an ultrasonic weld area.

Aspect 9. The sterilization wrap of any one or more of the preceding aspects, wherein the sterilization wrap is suitable for use to sterilize a medical device utilizing the simultaneous wrap technique during a sterilization process.

Aspect 10. The sterilization wrap of any one or more of the preceding aspects, wherein the sterilization wrap is suitable for use to sterilize a medical device without utilizing an additional trayliner during a sterilization process.

Aspect 11. The sterilization wrap of any one or more of the preceding aspects, wherein the wet-laid non-woven material comprises cellulose fibers, synthetic fibers, binder, and optionally a surfactant.

Aspect 12. The sterilization wrap of any one or more of the preceding aspects, wherein the total basis weight of the sterilization wrap is from about 70 grams per square meter (gsm) to about 170 gsm.

Aspect 13. The sterilization wrap of any one or more of the preceding aspects, wherein the inner layer is absorbent and moisture wicking.

Aspect 14. The sterilization wrap of any one or more of the preceding aspects, wherein the sterilization wrap is permeable to a sterilizing agent, such as steam.

Aspect 15. The sterilization wrap of any one or more of the preceding aspects, wherein the sterilization wrap exhibits microbial barrier properties and is impermeable to bacteria.

Aspect 16. The sterilization wrap of any one or more of the preceding aspects, wherein the sterilization wrap meets the requirements of European standard EN 868 Part 2 as a sterile barrier system.

Aspect 17. The sterilization wrap of any one or more of the preceding aspects, wherein the sterilization wrap meets the requirements of international standard ISO 11607 Part 1 as a sterile barrier system.

Aspect 18. The sterilization wrap of any one or more of the preceding aspects, wherein the basis weight of the outer layer is from about 35 grams per square meter (gsm) to about 85 gsm; and/or wherein the basis weight of the inner layer is from about 35 grams per square meter (gsm) to about 85 gsm.

Aspect 19. The sterilization wrap of any one or more of the preceding aspects, wherein the inner layer comprises cellulose fibers in a quantity between 40% to 100% by weight based on the dry weight of the fibers in the inner layer, preferably between 60% to 85% by weight based on the dry weight of the fibers in the inner layer; and wherein the cellulose fibers comprise at least one of hardwood fibers, softwood fibers, annual plant fibers or cotton fibers.

Aspect 20. The sterilization wrap of any one or more of the preceding aspects, wherein the inner layer comprises at least one of mercerized or un-mercerized cellulosic fibers, or a combination thereof.

Aspect 21. The sterilization wrap of any one or more of the preceding aspects, wherein the inner layer is micrexed or micro-creped.

Aspect 22. The sterilization wrap of any one or more of the preceding aspects, wherein the inner layer comprises synthetic fibers, preferably polyester staple fibers, more preferably polyethylene terephthalate (PET) fibers; and wherein the inner layer comprises synthetic fiber in a quantity between 5% to 60% by weight synthetic fibers, preferably 15% to 30%, the percentage based on the dry weight of the fibers of the inner layer, and optional synthetic binder fibers are included in this percentage, if present.

Aspect 23. The sterilization wrap of any one or more of the preceding aspects, wherein the inner layer comprises a binder, preferably comprising at least one of anionic acrylate-styrene and/or anionic acrylate-styrene-acrylonitrile, more preferably the binder comprising acrylic dispersions having acrylonitrile.

Aspect 24. The sterilization wrap of any one or more of the preceding aspects, wherein the inner layer comprises binder in a quantity between 15 wt% to 40 wt%, preferably 15 to 30 wt%, more preferably 18 to 28 wt%, even more preferably 20 to 25 wt%, the weight percentage based on the total dry weight of the inner layer.

Aspect 25. The sterilization wrap of any one or more of the preceding aspects, wherein the inner layer comprises a surfactant, the surfactant an anionic surfactant, preferably an anionic surfactant such as ammonium lauryl sulfate, sodium laureth sulfate, sodium lauryl sarcosinate, sodium myreth sulfate, sodium pareth sulfate, sodium stearte, sodium lauryl sulfate, α olefin sulfonate, and ammonium laureth sulfate, more preferably sodium bis(2-ethyl-1-hexyl) sulfosuccinate; and wherein the inner layer comprises surfactant in a quantity between 0.5 to 5%, preferably between 1 to 3%, more preferably between 1 to 2%, of a binder mixture.

Aspect 26. The sterilization wrap of any one or more of the preceding aspects, wherein the outer layer secured and bonded to the inner layer by at least the first weld area has a bond strength greater than or equal to 80 cN/15 mm, preferably greater than or equal to 100 cN/15 mm, according to the standard method ASTM F88.

Aspect 27. The sterilization wrap of any one or more of the preceding aspects, wherein at least one of the first weld area or the second weld area includes a plurality of continuous and/or non-continuous welding lines and/or welding spots.

Aspect 28. The sterilization wrap of any one or more of the preceding aspects, wherein the outer layer is treated with an anti-static composition.

Aspect 29. A method for fabricating a sterilization wrap comprising providing an outer layer comprising a thermoplastic material; and providing an inner layer comprising a wet-laid non-woven material; and securing and bonding the outer layer to the inner layer by at least a first weld area.

Aspect 30. The method of Aspect 29, wherein the outer layer is directly secured and directly bonded to the inner layer by at least the first weld area.

Aspect 31. The method of any one of Aspect 29 or Aspect 30, wherein the outer layer defines a thickness of thermoplastic material, and the inner layer defines a thickness of wet-laid non-woven material; and wherein the outer layer is not secured or bonded to another layer or thickness of material after the outer layer is secured and bonded to the inner layer by at least the first weld area.

Aspect 32. The method of any one of Aspects 29 to 31 further comprising securing and bonding the outer layer to the inner layer by at least the first weld area and by at least a second weld area, wherein the outer layer is secured and bonded to the inner layer by at least the first weld area along a first edge of the outer and inner layers, and wherein the outer layer is secured and bonded to the inner layer by at least the second weld area along a second edge of the outer and inner layers.

Aspect 33. The method of any one of Aspects 29 to 32, wherein the outer layer and the inner layer are each free of an added adhesive material.

Aspect 34. The method of any one of Aspects 29 to 33, wherein the outer layer comprises a non-woven melt-blown material, preferably wherein the outer layer comprises melt-blown synthetic fibers, more preferably wherein the outer layer comprises spunbond-meltblown-spunbond (SMS) synthetic fibers, even more preferably wherein the outer layer comprises SMS polyolefin fibers, such as SMS polypropylene fibers

Aspect 35. The method of any one of Aspects 29 to 34, wherein at least one of the first weld area or the second weld area is an ultrasonic weld.

Aspect 36. The method of any one of Aspects 29 to 35 further comprising utilizing the sterilization wrap to sterilize a medical device via the simultaneous wrap technique and without utilizing an additional trayliner during a sterilization process.

Aspect 37. The method of any one of Aspects 29 to 36, wherein the wet-laid non-woven material comprises cellulose, synthetic fibers, binder, and optionally a surfactant.

Aspect 38. The method of any one of Aspects 29 to 37, wherein the outer layer secured and bonded to the inner layer by at least the first weld area has a bond strength greater than or equal to 80 cN/15 mm, preferably greater than or equal to 100 cN/15 mm, according to the standard method ASTM F88.

While particular embodiments have been described, alternatives, modifications, variations, improvements, and substantial equivalents that are or may be presently unforeseen may arise to applicants or others skilled in the art. Accordingly, the appended claims as filed and as they may be amended are intended to embrace all such alternatives, modifications variations, improvements, and substantial equivalents.

All ranges disclosed herein are inclusive of the endpoints, and the endpoints are independently combinable with each other (e.g., ranges of "up to 25 wt.%, or, more specifically, 5 wt.% to 20 wt.%", is inclusive of the endpoints and all intermediate values of the ranges of "5 wt.% to 25 wt.%," etc.). "Combinations" is inclusive of blends, mixtures, alloys, reaction products, and the like. The terms "first," "second," and the like, do not denote any order, quantity, or importance, but rather are used to distinguish one element from another. The terms "a" and "an" and "the" do not denote a limitation of quantity and are to be construed to cover both the singular and the plural, unless otherwise indicated herein or clearly contradicted by context. "Or" means "and/or" unless clearly stated otherwise. Reference throughout the specification to "some embodiments", "an embodiment", and so forth, means that a particular element described in connection with the embodiment is included in at least one embodiment described herein, and may or may not be present in other embodiments. In addition, it is to be understood that the described elements may be combined in any suitable manner in the various embodiments. A "combination thereof" is open and includes any combination comprising at least one of the listed components or properties optionally together with a like or equivalent component or property not listed.

Unless defined otherwise, technical and scientific terms used herein have the same meaning as is commonly understood by one of skill in the art to which this application belongs. All cited patents, patent applications, and other references are incorporated herein by reference in their entirety. However, if a term in the present application contradicts or conflicts with a term in the incorporated reference, the term from the present application takes precedence over the conflicting term from the incorporated reference.

Unless specified to the contrary herein, all test standards are the most recent standard in effect as of the filing date of this application, or, if priority is claimed, the filing date of the earliest priority application in which the test standard appears.

Although the sterilization wraps, systems and methods of the present disclosure have been described with reference to exemplary embodiments thereof, the present disclosure is not limited to such exemplary embodiments and/or implementations. Rather, the sterilization wraps, systems and methods of the present disclosure are susceptible to many implementations and applications, as will be readily apparent to persons skilled in the art from the disclosure hereof. The present disclosure expressly encompasses such modifications, enhancements and/or variations of the disclosed embodiments. Since many changes could be made in the above construction and many widely different embodiments of this disclosure could be made without departing from the scope thereof, it is intended that all matter contained in the drawings and specification shall be interpreted as illustrative and not in a limiting sense. Additional modifications, changes, and substitutions are intended in the foregoing disclosure. Accordingly, it is appropriate that the appended claims be construed broadly and in a manner consistent with the scope of the disclosure.

### PREFERRED EMBODIMENTS

1. A sterilization wrap comprising:
   an outer layer comprising a thermoplastic material; and
   an inner layer comprising a wet-laid non-woven material;
   wherein the outer layer is secured and bonded to the inner layer by at least a first weld area.
2. The sterilization wrap of embodiment 1, wherein the outer layer is directly secured and directly bonded to the inner layer by at least the first weld area.
3. The sterilization wrap of any one or more of the preceding embodiments,
   wherein the outer layer defines a thickness of thermoplastic material, and the inner layer defines a thickness of wet-laid non-woven material; and
   wherein the outer layer is not secured or bonded to another layer or thickness of material after the outer layer is secured and bonded to the inner layer by at least the first weld area.
4. The sterilization wrap of any one or more of the preceding embodiments,
   wherein the outer layer is secured and bonded to the inner layer by at least the first weld area along a first edge of the outer and inner layers.
5. The sterilization wrap of embodiment 4, wherein the outer layer is secured and bonded to the inner layer by at least a second weld area along a second edge of the outer and inner layers.
6. The sterilization wrap of any one or more of the preceding embodiments,
   wherein the outer layer and the inner layer are each free of an added adhesive material.
7. The sterilization wrap of any one or more of the preceding embodiments,
   wherein the outer layer comprises a non-woven melt-blown material, preferably wherein the outer layer comprises melt-blown synthetic fibers, more preferably wherein the outer layer comprises spunbond-meltblown-spunbond (SMS) synthetic fibers, even more preferably wherein the outer layer comprises SMS polyolefin fibers, such as SMS polypropylene fibers.
8. The sterilization wrap of embodiment 5, wherein at least one of the first weld area or the second weld area is an ultrasonic weld area.
9. The sterilization wrap of any one or more of the preceding embodiments,
   wherein the sterilization wrap is suitable for use to sterilize a medical device utilizing the simultaneous wrap technique during a sterilization process.
10. The sterilization wrap of any one or more of the preceding embodiments,
   wherein the sterilization wrap is suitable for use to sterilize a medical device without utilizing an additional trayliner during a sterilization process.
11. The sterilization wrap of any one or more of the preceding embodiments,
   wherein the wet-laid non-woven material comprises cellulose fibers, synthetic fibers, binder, and optionally a surfactant.
12. The sterilization wrap of any one or more of the preceding embodiments,
   wherein the total basis weight of the sterilization wrap is from about 70 grams per square meter (gsm) to about 170 gsm.
13. The sterilization wrap of any one or more of the preceding embodiments,
   wherein the inner layer is absorbent and moisture wicking.
14. The sterilization wrap of any one or more of the preceding embodiments,
   wherein the sterilization wrap is permeable to a sterilizing agent, such as steam.
15. The sterilization wrap of any one or more of the preceding embodiments,
   wherein the sterilization wrap exhibits microbial barrier properties and is impermeable to bacteria.
16. The sterilization wrap of any one or more of the preceding embodiments,
   wherein the sterilization wrap meets the requirements of European standard EN 868 Part 2 as a sterile barrier system.
17. The sterilization wrap of any one or more of the preceding embodiments,
   wherein the sterilization wrap meets the requirements of international standard ISO 11607 Part 1 as a sterile barrier system.
18. The sterilization wrap of any one or more of the preceding embodiments,
   wherein the basis weight of the outer layer is from about 35 grams per square meter (gsm) to about 85 gsm; and/or
   wherein the basis weight of the inner layer is from about 35 grams per square meter (gsm) to about 85 gsm.
19. The sterilization wrap of any one or more of the preceding embodiments,
   wherein the inner layer comprises cellulose fibers in a quantity between 40% to 100% by weight based on the dry weight of the fibers in the inner layer, preferably between 60% to 85% by weight based on the dry weight of the fibers in the inner layer;
   and wherein the cellulose fibers comprise at least one of hardwood fibers, softwood fibers, annual plant fibers or cotton fibers.
20. The sterilization wrap of any one or more of the preceding embodiments,
   wherein the inner layer comprises at least one of mercerized or un-mercerized cellulosic fibers, or a combination thereof.
21. The sterilization wrap of any one or more of the preceding embodiments,
   wherein the inner layer is micrexed or micro-creped.
22. The sterilization wrap of any one or more of the preceding embodiments,
   wherein the inner layer comprises synthetic fibers, preferably polyester staple fibers, more preferably polyethylene terephthalate (PET) fibers; and
   wherein the inner layer comprises synthetic fiber in a quantity between 5% to 60% by weight synthetic fibers, preferably 15% to 30%, the percentage based on the dry weight of the fibers of the inner layer, and optional synthetic binder fibers are included in this percentage, if present.
23. The sterilization wrap of any one or more of the preceding embodiments,
   wherein the inner layer comprises a binder, preferably comprising at least one of anionic acrylate-styrene and/or anionic acrylate-styrene-acrylonitrile, more preferably the binder comprising acrylic dispersions having acrylonitrile.
24. The sterilization wrap of any one or more of the preceding embodiments,
   wherein the inner layer comprises binder in a quantity between 15 wt% to 40 wt%, preferably 15 to 30 wt%, more preferably 18 to 28 wt%, even more preferably 20 to 25 wt%, the weight percentage based on the total dry weight of the inner layer.
25. The sterilization wrap of any one or more of the preceding embodiments,
   wherein the inner layer comprises a surfactant, the surfactant an anionic surfactant, preferably an anionic surfactant such as ammonium lauryl sulfate, sodium laureth sulfate, sodium lauryl sarcosinate, sodium myreth sulfate, sodium pareth sulfate, sodium stearte, sodium lauryl sulfate, α olefin sulfonate, and ammonium laureth sulfate, more preferably sodium bis(2-ethyl-1-hexyl) sulfosuccinate; and
   wherein the inner layer comprises surfactant in a quantity between 0.5 to 5%, preferably between 1 to 3%, more preferably between 1 to 2%, of a binder mixture.
26. The sterilization wrap of any one or more of the preceding embodiments,
   wherein the outer layer secured and bonded to the inner layer by at least the first weld area has a bond strength greater than or equal to 80 cN/15 mm, preferably greater than or equal to 100 cN/15 mm, according to the standard method ASTM F88.
27. The sterilization wrap of embodiment 5, wherein at least one of the first weld area or the second weld area includes a plurality of continuous and/or non-continuous welding lines and/or welding spots.
28. The sterilization wrap of any one or more of the preceding embodiments,
   wherein the outer layer is treated with an anti-static composition.
29. A method for fabricating a sterilization wrap comprising:
   providing an outer layer comprising a thermoplastic material; and
   providing an inner layer comprising a wet-laid non-woven materialand securing and bonding the outer layer to the inner layer by at least a first weld area.
30. The method of embodiment 29, wherein the outer layer is directly secured and directly bonded to the inner layer by at least the first weld area.
31. The method of any one of embodiment 29 or embodiment 30, wherein the outer layer defines a thickness of thermoplastic material, and the inner layer defines a thickness of wet-laid non-woven material; and
   wherein the outer layer is not secured or bonded to another layer or thickness of material after the outer layer is secured and bonded to the inner layer by at least the first weld area.
32. The method of any one of embodiments 29 to 31 further comprising securing and bonding the outer layer to the inner layer by at least the first weld area and by at least a second weld area, wherein the outer layer is secured and bonded to the inner layer by at least the first weld area along a first edge of the outer and inner layers, and wherein the outer layer is secured and bonded to the inner layer by at least the second weld area along a second edge of the outer and inner layers.
33. The method of any one of embodiments 29 to 32, wherein the outer layer and the inner layer are each free of an added adhesive material.
34. The method of any one of embodiments 29 to 33, wherein the outer layer comprises a non-woven melt-blown material, preferably wherein the outer layer comprises melt-blown synthetic fibers, more preferably wherein the outer layer comprises spunbond-meltblown-spunbond (SMS) synthetic fibers, even more preferably wherein the outer layer comprises SMS polyolefin fibers, such as SMS polypropylene fibers.
35. The method of embodiment 32, wherein at least one of the first weld area or the second weld area is an ultrasonic weld.
36. The method of any one of embodiments 29 to 35 further comprising utilizing the sterilization wrap to sterilize a medical device via the simultaneous wrap technique and without utilizing an additional trayliner during a sterilization process.
37. The method of any one of embodiments 29 to 36, wherein the wet-laid non-woven material comprises cellulose, synthetic fibers, binder, and optionally a surfactant.
38. The method of any one of embodiments 29 to 37, wherein the outer layer secured and bonded to the inner layer by at least the first weld area has a bond strength greater than or equal to 80 cN/15 mm, preferably greater than or equal to 100 cN/15 mm, according to the standard method ASTM F88.

## Claims

1. A sterilization wrap comprising:
an outer layer comprising a thermoplastic material; and
an inner layer comprising a wet-laid non-woven material;
wherein the outer layer is directly secured and directly bonded to the inner layer by at least a first weld area.

2. The sterilization wrap of claim 1, wherein the outer layer defines a thickness of thermoplastic material, and the inner layer defines a thickness of wet-laid non-woven material; and
wherein the outer layer is not secured or bonded to another layer or thickness of material after the outer layer is secured and bonded to the inner layer by at least the first weld area.

3. The sterilization wrap of any one or more of the preceding claims, wherein the outer layer is secured and bonded to the inner layer by at least the first weld area along a first edge of the outer and inner layers.

4. The sterilization wrap of Claim 3, wherein the outer layer is secured and bonded to the inner layer by at least a second weld area along a second edge of the outer and inner layers.

5. The sterilization wrap of any one or more of the preceding claims, wherein the outer layer comprises a non-woven melt-blown material, preferably wherein the outer layer comprises melt-blown synthetic fibers, more preferably wherein the outer layer comprises spunbond-meltblown-spunbond (SMS) synthetic fibers, even more preferably wherein the outer layer comprises SMS polyolefin fibers, such as SMS polypropylene fibers.

6. The sterilization wrap of any one or more of the preceding claims, wherein the sterilization wrap is permeable to a sterilizing agent, such as steam and/or wherein the sterilization wrap exhibits microbial barrier properties and is impermeable to bacteria.

7. The sterilization wrap of any one or more of the preceding claims, wherein the inner layer comprises cellulose fibers in a quantity between 40% to 100% by weight based on the dry weight of the fibers in the inner layer, preferably between 60% to 85% by weight based on the dry weight of the fibers in the inner layer;
and wherein the cellulose fibers comprise at least one of hardwood fibers, softwood fibers, annual plant fibers or cotton fibers.

8. The sterilization wrap of any one or more of the preceding claims, wherein the inner layer comprises synthetic fibers, preferably polyester staple fibers, more preferably polyethylene terephthalate (PET) fibers; and
wherein the inner layer comprises synthetic fiber in a quantity between 5% to 60% by weight synthetic fibers, preferably 15% to 30%, the percentage based on the dry weight of the fibers of the inner layer, and wherein when the binder comprises synthetic binder fibers, the synthetic binder fibers are included in this percentage.

9. The sterilization wrap of any one or more of the preceding claims, wherein the inner layer comprises a binder, preferably comprising at least one of anionic acrylate-styrene and/or anionic acrylate-styrene-acrylonitrile, more preferably the binder comprising acrylic dispersions having acrylonitrile.

10. The sterilization wrap of any one or more of the preceding claims, wherein the inner layer comprises binder in a quantity between 15 wt% to 40 wt%, preferably 15 to 30 wt%, more preferably 18 to 28 wt%, even more preferably 20 to 25 wt%, the weight percentage based on the total dry weight of the inner layer.

11. The sterilization wrap of any one or more of the preceding claims, wherein the inner layer comprises a surfactant, the surfactant being an anionic surfactant, preferably an anionic surfactant such as ammonium lauryl sulfate, sodium laureth sulfate, sodium lauryl sarcosinate, sodium myreth sulfate, sodium pareth sulfate, sodium stearte, sodium lauryl sulfate, α olefin sulfonate, and ammonium laureth sulfate, more preferably sodium bis(2-ethyl-1-hexyl) sulfosuccinate; and
wherein the inner layer comprises surfactant in a quantity between 0.5 to 5%, preferably between 1 to 3%, more preferably between 1 to 2%, of a binder mixture.

12. The sterilization wrap of Claim 4, wherein at least one of the first weld area or the second weld area includes a plurality of continuous and/or non-continuous welding lines and/or welding spots.

13. A method for fabricating a sterilization wrap comprising:
providing an outer layer comprising a thermoplastic material; and
providing an inner layer comprising a wet-laid non-woven material and
securing and bonding the outer layer to the inner layer by at least a first weld area;
wherein the outer layer is directly secured and directly bonded to the inner layer by at least the first weld area.

14. The method of Claim 13 further comprising securing and bonding the outer layer to the inner layer by at least the first weld area and by at least a second weld area, wherein the outer layer is secured and bonded to the inner layer by at least the first weld area along a first edge of the outer and inner layers, and wherein the outer layer is secured and bonded to the inner layer by at least the second weld area along a second edge of the outer and inner layers.

15. The method of Claim 14, wherein at least one of the first weld area or the second weld area is an ultrasonic weld.
